# EUROPÄISCHE PATENTSCHRIFT

(11) **EP 0 637 312 B1**
(45) Veröffentlichungstag und Bekanntmachung des Hinweises auf die Patenterteilung: **05.03.1997**
(21) Anmeldenummer: 93909366.2
(22) Anmeldetag: 08.04.1993
(51) Int. Cl.: C07H 15/04, C11D 1/66, C11D 1/83

(54) **VERFAHREN ZUR HERSTELLUNG VON ALKYLGLYKOSIDEN UND IHRE VERWENDUNG**
METHOD FOR THE PREPARATION OF ALKYL GLYCOSIDES AND THEIR USE
PROCEDE DE PRODUCTION D'ALKYLGLUCOSIDES ET LEUR UTILISATION

(30) Priorität: 21.04.1992 DE 4213016
(43) Veröffentlichungstag der Anmeldung: 08.02.1995
(73) Patentinhaber: BASF Aktiengesellschaft, 67063 Ludwigshafen (DE)
(72) Erfinder: WOLF, Gerhard, D-6800 Mannheim 24 (DE); OFTRING, Alfred, D-6702 Bad Duerkheim (DE); OETTER, Guenter, D-6710 Frankenthal (DE); SCHMIDT, Richard, R., D-7750 Konstanz 16 (DE); KLOTZ, Wolfgang, D-7750 Konstanz (DE)
(86) Internationale Anmeldenummer: EP9300875
(87) Internationale Veröffentlichungsnummer: WO9321197

(56) Entgegenhaltungen:
- EP-A- 0 364 852
- WO-A-92/02604
- DE-B- 1 183 894
- US-A- 4 663 444
- CHEMICAL ABSTRACTS, vol. 98, 1983, Columbus, Ohio, US, abstract no. 179770, SZEJA W.

## Beschreibung

Die Erfindung betrifft die Herstellung von Alkylglykosiden durch Alkylierung von reduzierenden Sacchariden mit Dialkylsulfaten in Gegenwart einer Base und eines Lösemittels.

Oberflächenaktive Verbindungen auf Basis von nachwachsenden Rohstoffen gewinnen zunehmend an Bedeutung. Bei den anionischen Tensiden wären hier z.B. die Fettalkoholsulfate und alpha-Sulfofettsäuremethylester zu nennen, während bei den nichtionischen Tensiden Kohlehydratfettsäureester, Fettalkylglukamide und insbesondere Alkylglykoside zu erwähnen sind. Alkylglykoside sind als oberflächenaktive Kohlehydrat-Tenside seit langem bekannt, werden aber wegen ihrer relativ aufwendigen Herstellung bisher nur begrenzt in Wasch- und Reinigungsmitteln eingesetzt. Alkylgykoside können nach der klassischen Königs-Knorr-Methode über die Aktivierung der C-1-Position aus den entsprechenden Halogenesen und langkettigen Alkoholen hergestellt werden. Eine Fülle an weiteren, meist aufwendigen Glykosilierungs-Varianten sind in der Literatur beschrieben, vgl. P. Sinay, Pure & Appl. Chem., Vol. 63, 519, (1991) und R.R. Schmidt, Angew. Chem. Band 98, 213 (1986).

Alkylglykoside werden in der Technik prinzipiell nach 2 verschiedenen Verfahren hergestellt. Bei der sog. Umacetalisierungsmethode wird zunächst in einer 1. Verfahrensstufe eine hydrophile Monosaccharid-Einheit mit einem kurzkettigen Alkohol (meistens n-Butanol oder Glykol) unter Säure-Katalyse zu einem kurzkettigen Alkylglykosid umgesetzt und dann in einer 2. Stufe durch Reaktion mit einem langkettigen Alkohol (Umacetalisierung) zum oberflächenaktiven Alkylglykosid umgesetzt. Bei einem anderen gebräuchlichen Verfahren wird eine Monosaccharid-Einheit direkt unter Säure-Katalyse mit dem langkettigen, hydrophoben Alkohol zur Reaktion gebracht. Beide Herstell-Verfahren sind in der Patent-Literatur in vielerlei Varianten beschrieben, z.B. US-A-3 547 828, US-A-3 598 865, US-A-3 839 318, EP-A-0 362 671 und EP-A-0 252 241.

Da es sich bei den beiden oben beschriebenen Verfahren der Glykosilierung um eine Gleichgewichtsreaktion handelt, wird die Alkoholkomponente jeweils im Überschuß eingesetzt. Nach der Reaktion muß der im Überschuß eingesetzte Alkohol aus dem Reaktionsprodukt entfernt werden. Die destillative Abtrennung von langkettigen Alkoholen aus dem Reaktionsgemisch ist technisch aufwendig und teuer. Hierbei kommt es außerdem aufgrund der thermischen Labilität der Alkylglykoside zu starken Farbschädigungen des Reaktionsprodukts. Die so erhältlichen Alkylglykoside sind Gemische von Alkylmonoglykosiden und Alkyloligo- bzw. -polyglykosiden.

Die Per-alkylierung von Kohlehydraten mit Dimethylsulfat ist seit langem bekannt, vgl. Houben-Weyl, Band 6/3, 35f (1965). Auch die selektive Methylierung von Glykose in der C-1-Position mit Dimethylsulfat ist in der Literatur beschrieben, vgl. D.M. Hall und O.A. Stamm, Carbohydr. Res., Band 12, 412 (1970).

Aus der US-A 4 663 444 ist bekannt, zur Herstellung von in der C-6-Position alkylierten oder arylierten Glucosiden 1-Alkylglucoside mit Methylmesylaten der Formel R¹-O-SO₂CH₃ (R¹= Aryl, Aralkyl, Alkyl oder Alkenyl mit 1 bis 20 C-Atomen) in Gegenwart von Basen wie KOH und von Lösungsmitteln wie Dimethylsulfoxid umzusetzen.

Die EP-A 364 852 offenbart ein Verfahren zur Herstellung von substituierten Glucosiden mit C₁- bis C₄-Alkylgruppen in der Etherbindung, wobei als Verbindungen mit nucleophiler Abgangsgruppe Dimethylsulfat bis Dibutylsulfat genannt sind. Als Ausgangsverbindung für die Alkylierung wird ein Glucosid mit einem C₈- bis C₁₈-Alkylrest in der Acetalbindung verwendet.

Chem. Abstr. 98 (1983), 179 770 offenbart ein Verfahren zur Herstellung von substituierten Glucosiden mit Alkylgruppen in der Etherbindung in einem Zweiphasen-System, wobei als einziges Beispiel das schon lang bekannte Dimethylsulfat als Alkyldonor erwähnt wird.

Der vorliegenden Erfindung liegt die Aufgabe zugrunde, ein verbessertes Verfahren zur Herstellung von Alkylglykosiden, die für den Einsatz in Waschmitteln geeignet sind, zur Verfügung zu stellen.

Die Aufgabe wird erfindungsgemäß gelöst mit einem Verfahren zur Herstellung von Alkylglykosiden durch Alkylierung von reduzierenden Sacchariden mit Alkylierungsmitteln in Gegenwart einer Base und eines Lösemittels, wenn man als Alkylierungsmittel Dialkylsulfate einsetzt, deren Alkylgruppen mindestens 6 C-Atome aufweisen und wenn das Molverhältnis von Sacchariden zu Dialkylsulfaten 1:1 bis 1:5 beträgt. Die so erhältlichen Reaktionsmischungen werden als Tenside verwendet, vorzugsweise in Wasch- und Reinigungsmitteln.

Die Umsetzung von reduzierenden Sacchariden mit Dialkylsulfaten läuft gemäß folgendem Schema ab: 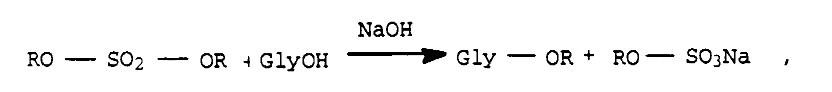 wobei GlyOH ein reduzierendes Saccharid und R eine Alkylgruppe bedeutet.

Sofern man die reduzierenden Saccharide und die Dialkylsulfate im Molverhältnis 1:1 einsetzt, erhält man praktisch äquivalente Mengen an Alkylmonoglykosiden und Salzen von Alkoholsulfaten. Bei der Umsetzung von Dialkylsulfaten mit reduzierenden Saccharid-Einheiten reagiert im wesentlichen nur eine Alkylgruppe des Dialkylsulfats. Nach der Neutralisation des Alkoholsulfats mit Basen, vorzugsweise Natronlauge, liegt bei Einsatz von Dialkylsulfaten mit mindestens 6 Kohlenstoffatomen in der Alkylgruppe ein anionisches Tensid vor. Die so erhältlichen Mischungen können daher anstelle sonst Üblicher Tenside in Wasch- und Reinigungsmitteln eingesetzt werden. Will man dagegen reine Alkylmonoglykoside herstellen, die vorwiegend als Tenside oder Emulgatoren verwendet werden, so muß das bei der oben angegebenen Reaktion von Dialkylsulfaten mit reduzierenden Saccharid-Einheiten entstehende Reaktionsgemisch gereinigt werden.

Die eingesetzten Dialkylsulfate mit mindestens 6 C-Atomen in der Alkylgruppe lassen sich leicht aus den entsprechenden Alkoholen gleicher Kohlenstoffzahl und beispielsweise Sulfurylchlorid herstellen. Die in Betracht kommenden Dialkylsulfate können beispielsweise mit Hilfe folgender allgemeiner Formel charakterisiert werden

R-O-SO₂-O-R¹ (I),

in der R und R¹ gleiche oder verschiedene Reste sein können und für C₆- bis C₃₀-Alkyl oder C₆- bis C₃₀-Alkenyl stehen. Von technischem Interesse sind vor allem solche Verbindungen der Formel I, in der R, R¹ für C₆- bis C₃₀-, vorzugsweise C₈- bis C₁₈-Alkyl oder Alkenyl stehen.

In der oben angegebenen Formel I können die Substituenten R und R¹ prinzipiell unterschiedliche Kohlenstoff-Kettenlängen besitzen. Bei Einsatz dieser Dialkylsulfate entstehen dann bei der Umsetzung mit reduzierenden Sacchariden Mischungen aus zwei Alkylglykosiden und Mischungen aus zwei Alkoholsulfaten. Vorzugsweise bedeutet in der oben angegebenen Formel I R = R¹.

Die aliphatischen Alkohole, die zur Bildung der Dialkylsulfate eingesetzt werden, können an sich beliebige Kettenlängen d.h. solche von 6 bis etwa 30 Kohlenstoffatomen, aufweisen. Um oberflächenaktive Reaktionsprodukte, die als Tensidrohstoffe in Wasch- und Reinigungsmitteln eingesetzt werden können, zu erhalten, werden aliphatische primäre Alkohole mit 6 bis 20 Kohlenstoffatomen, insbesondere solche mit 8 bis 18 Kohlenstoffatomen, bevorzugt. Diese höheren aliphatischen Alkohole werden vorzugsweise aus technischen Fetten hergestellt. Selbstverständlich ist es aber auch möglich, synthetische primäre Alkohole wie z.B. die sogenannten Oxoalkohole zur Herstellung der Dialkylsulfate einzusetzen. Die Alkylgruppen der vorzugsweise eingesetzten Dialkylsulfate enthalten 6 bis 30 C-Atome und weisen gegebenenfalls eine Doppelbindung auf.

Bei den als Alkoholkomponente besonders wichtigen höheren aliphatischen primären C₁₂- bis C₁₈-Alkoholen zur Herstellung der Dialkylsulfate handelt es sich vorzugsweise um gesättigte und insbesondere um geradkettige Alkohle, wie sie durch die Hydrierung von nativen Fettsäuren im technischen Maßstab erhalten werden können, z.B. die Verbindungen n-Dodecylalkohol, n-Tetradecylalkanol, n-Hexadecylalkohol, n-Octadecylalkohol, n-Octylalkohol, n-Decylalkohol, Undecylalkohol und Tridecylalkohol. Da die Fettalkohole bevorzugt aus natürlichen Fettquellen stammen, kommen üblicheweise auch Gemische technischer Fettalkohole als Reaktionspartner in Betracht. Neben den eigentlichen Fettalkoholen sind auch verzweigtkettige primäre Alkohole, wie z.B. die sogenannten Oxoalkohole für die Umsetzung geeignet. Typische Oxoalkohole sind z.B. die Verbindungen C₁₂-C₁₃-Alkanol mit ca. 25 % hauptsächlich 2-Methylverzweigung (Dobanol 23) und der entsprechende C₉-C₁₁-Alkanol (Dobanol 91).

Die reduzierenden Saccharide können Mono-, Di- und Oligosaccharide oder Gemische derselben sein und sind aus üblichen Pentosen und Hexosen aufgebaut. Als derartige Bausteine dienen Aldopentosen wie z.B. Ribose, Arabinose, Xylose und Lyxose, Aldohexosen wie z.B. Allose, Altrose, Glucose, Mannose, Gulose, Idose, Galactose und Talose, wie Kethohexosen wie Fructose. Bevorzugt werden Mannose, Glucose, Galactose und Fructose, besonders bevorzugt Glucose.

Als Disaccharide können vor allem Saccharose, Lactose, Maltose und Cellobiose verwendet werden. Zudem ist es möglich, die Zuckerkomponente sowohl in wasserfreier Form, als Monohydrat oder als Sirup einzusetzen. Bei letzterem kann der Wassergehalt der Saccharid-Lösungen zwischen 10 und 80 Gew.-% liegen. Bevorzugt werden technische Dextrosesirupe, die einen Wassergehalt von 20 bis 40 Gew.-% haben.

Die Reaktion von Dialkylsulfaten mit den reduzierenden Sacchariden wird in Gegenwart einer Base durchgeführt. Die Basen deprotonieren die Hydroxylgruppe in der C-1-Position der Monosaccharid-Einheit. Sie werden in mindestens äquimolarem Verhältnis eingesetzt. Das Molverhältnis von Base zu Monosaccharid beträgt 1:1 bis 10:1, vorzugsweise 1:1 bis 5:1. In den meisten Fällen beträgt das Molverhältnis von eingesetzter Base zu Monosaccharid 1:1 bis 2:1. Als Basen werden Hydroxyde, Amide, Hydride, Oxyde oder Carbonate von Alkalimetallen und/oder Erdalkalimetallen, Borhydrid, Natriumborhydrid oder Hydride, Hydroxide, Oxide oder Carbonate des Aluminiums eingesetzt. Bevorzugt eingesetzte Basen sind Natronlauge, Kalilauge, Natriumhydrid, Borhydrid, Natriumborhydrid, Lithiumhydrid und Lithiumaluminiumhydrid. Aufgrund des Basenzusatzes entstehen gemäß dem oben angegebenen Reaktionsschema die entsprechenden Salze des Schwefelsäurealkylmonoesters.

Das Molverhältnis von Sacchariden zu Dialkylsulfaten beträgt 1:1 bis 1:5. Falls die bei der Reaktion entstehende Mischung aus Alkylmonoglykosid und Alkoholsulfat direkt als Zusatz zu Wasch- und Reinigungsmitteln verwendet werden soll, empfiehlt es sich, bei der Herstellung der Produkte nur mit einem geringen Überschuß an Dialkylsulfat zu arbeiten, z.B. beträgt dann das Molverhältnis von Saccharid zu Dialkylsulfat 1:1 bis 1:1,05. In diesem Fall wird vorzugsweise Wasser als Lösemittel und Natronlauge oder Kalilauge als Base eingesetzt. Prinzipiell können auch andere Lösemittel verwendet werden, diese müssen jedoch mit Hilfe einer Destillation oder Chromatographie von den Reaktionsprodukten abgetrennt werden können. Wasser hat gegenüber den anderen Lösemittel den Vorteil, daß es im Produkt verbleiben kann. Zur Steuerung der Reaktion ist es möglich, das Molverhältnis von Alkylmonoglykosiden zu Alkoholsulfaten in dem Bereich von 3:2 bis 2:3 zu variieren. Insbesondere werden Gemische aus C₆-bis C₃₀-Alkylmonoglykosiden und C₆- bis C₃₀-Alkoholsulfaten im molaren Verhältnis von 3:2 bis 2:3 als Tenside in Waschund Reinigungsmitteln eingesetzt.

Bevorzugt eingesetztes Lösemittel bei der Umsetzung von reduzierenden Sacchariden mit Dialkylsulfaten ist Wasser. Daneben eignen sich sowohl polare aprotische als auch polare protische Lösemittel, beispielsweise Hexamethylphosphorsäuretriamid, Dimethylformamid, N-Methylpyrrolidon, Dimethylproylenharnstoff, Dimethylethylenharnstoff, Dimethoxyethan, Tetrahydrofuran, Dioxan und Kohlendioxid. In manchen Fällen kann es von Vorteil sein, Gemische von Lösemitteln zu verwenden. Die Menge an eingesetzten Lösemitteln ist im allgemeinen nicht kritisch, üblicherweise verwendet man die Lösemittel in Mengen von 20 bis 90, vorzugsweise 30 bis 70 Gew.-%, bezogen auf die eingesetzten reduzierenden Saccharide und Dialkylsulfate. Da in Wasch- und Reinigungsmitteln bevorzugt gleichzeitig nichtionische und anionische Tenside eingesetzt werden, wobei zumeist die anionischen Tenside überwiegen, kann das bei der Umsetzung von reduzierenden Sacchariden und Dialkylsulfaten erhaltene Reaktionsgemisch leicht in übliche Wasch- und Reinigungsmittelformulierungen eingearbeitet werden. Das Reaktionsgemisch kann problemlos mit zusätzlichen anderen anionischen Tensiden gemischt werden. Dabei entstehen stabile Formulierungen. Geeignete Zusätze für die bei dem erfindungsgemäßen Verfahren anfallenden Reaktionsmischungen sind Arylsulfat, Alkoholethersulfate und Alkoholsulfate.

Für die Herstellung der reinen Alkylglykoside unter Abtrennung der Alkoholsulfate aus dem Reaktionsgemisch haben sich besonders Dimethylpropylenharnstoff und Natriumhydrid bewährt. Hierbei kann praktischerweise bei Temperaturen von 0 bis 100, bevorzugt 20 bis 30°C gearbeitet werden. Die Abtrennung des Lösemittels erfolgt mit Hilfe einer Vakuumdestillation, die Trennung von Alkylmonoglykosid und Alkoholsulfat wird vorzugsweise mit Hilfe der Chromatographie auf Reverse-Phase-Säulen (abgekürzt RP-Säulen) vorgenommen.

Die Umsetzung von Dialkylsulfaten mit reduzierenden Sacchariden kann auch in Gegenwart von Wasser oder der anderen Lösemittel in dem Temperaturbereich von 0 bis 100°C durchgeführt werden. Die Reaktionsmischungen, die als wesentliche Bestandteile Alkylmonoglykoside und Alkoholsulfate enthalten, werden als Tenside verwendet. Das oben beschriebene Verfahren erlaubt die Herstellung von reinen, oberflächenaktiven Alkylmonoglykosiden, ohne daß größere Mengen an unerwünschten Neben- und Abfallprodukten (z.B. Alkohole, Lösemittel oder höher alkylierte Saccharide) entstehen. Gegenüber den eingangs beschriebenen bekannten Herstellverfahren für Alkylglykoside ist es bei dem erfindungsgemäßen Verfahren möglich, bei sehr niedrigen Temperaturen zu arbeiten, so daß die erhaltenen Alkylmonoglykoside sehr hellfarbig sind und keiner zusätzlichen Reinigung, beispielsweise einer Bleichung, unterworfen werden müssen. Während bei den in der Technik ausgeübten Verfahren zur Herstellung von Alkylglykosiden Gemische aus Alkylmono-, Oligo-, und -polyglykosiden entstehen, erhält man bei dem erfindungsgemäßen Verfahren Alkylmonoglykoside.

### Beispiele

### Beispiel 1

90 g (0,5 mol) Glucose wurden in 400 ml absoluten Hexamethylphosphorsäuretriamid bei 20 bis 30°C gelöst; portionsweise wurden 20 g (0,75 mol) Natriumhydrid zugegeben. Nach beendeter Wasserstoff-Entwicklung wurden 284 g (0,75 mol) Didecylsulfat zugegeben und 15 h bei 20 bis 25°C gerührt. Überschüssiges Natriumhydrid wurde mit Methanol vernichtet und das Lösungsmittel am Hochvakuum (0,05 mbar) bei 110°C entfernt. Der Rückstand wurde in 100 ml Wasser/Methanol (3:7 Gewichtsteile) aufgenommen und über eine RP₁₈-Flash-Säule chromatographiert. Man erhielt als analysenreines Produkt 99,2 g Decylglucosid (62,5 % Ausbeute) mit einem alpha/ beta-Isomerenverhältnis von 1:2.

### Beispiel 2

135 g (0,75 mol) Galactose und 425 g (1,125 mol) Didecylsulfat wurden in 500 ml Dimethylpropylenharnstoff suspendiert, wobei die Ausgangsverbindungen teilweise in Lösung gingen. Bei 20 bis 30°C wurden 28 g (1,125 mol) Natriumhydrid zugegeben und 18 h gerührt. Das überschüssige Natriumhydrid wurde mit Methanol vernichtet und die hellgelbe Lösung am Hochvakuum (0,05 mbar) bei 100°C vom Lösemittel befreit. Analog zur Aufarbeitung in Beispiel 1 erhielt man 198 g Decyl-alpha-D-galactopyranosid (83 % Ausbeute).

### Beispiel 3

Analog Beispiel 2 erhielt man mit Glucose 179 g Decylglucosid (74 % Ausbeute) mit einem alpha/beta-Isomerenverhältnis von 1:3.

## Patentansprüche

1. Verfahren zur Herstellung von Alkylglykosiden durch Alkylierung von reduzierenden Sacchariden mit Alkylierungsmitteln in Gegenwart einer Base und eines Lösemittels, dadurch gekennzeichnet, daß man als Alkylierungsmittel Dialkylsulfate einsetzt, deren Alkylgruppen mindestens 6 C-Atome aufweisen und daß das Molverhältnis von Sacchariden zu Dialkylsulfaten 1:1 bis 1:5 beträgt.

2. Verfahren nach Anspruch 1, dadurch gekennzeichnet, daß die Alkylgruppen der Dialkylsulfate 6 bis 30 C-Atome aufweisen und gegebenenfalls eine Doppelbindung enthalten.

3. Verfahren nach Anspruch 1 oder 2, dadurch gekennzeichnet, daß man als Basen Hydroxide, Amide, Hydride, Oxide oder Carbonate von Alkalimetallen und/oder Erdalkalimetallen, Borhydrid, Natriumborhydrid oder Hydride, Hydroxide, Oxide oder Carbonate des Aluminiums einsetzt.

4. Verfahren nach einem der Ansprüche 1 bis 3, dadurch gekennzeichnet, daß das Molverhältnis der eingesetzten Basen zu den Saccharid-Einheiten 1:1 bis 10:1 beträgt.

## Claims

1. A process for preparing alkylglycosides by alkylation of reducing saccharides in the presence of a base and a solvent, characterized in that the alkylating agent used is a dialkyl sulfate whose alkyl groups contain at least 6 carbon atoms, the molar ratio of saccharide to dialkyl sulfate being from 1:1 to 1:5.

2. A process as claimed in claim 1, wherein the alkyl groups of the dialkyl sulfate contain 6 to 30 carbon atoms and may contain a double bond.

3. A process as claimed in claim 1 or 2, wherein the base used is a hydroxide, amide, hydride, oxide or carbonate of an alkali metal and/or alkaline earth metal, boron hydride, sodium borohyride or an hydride, hydroxide, oxide or carbonate of aluminum.

4. A process as claimed in claim 1 or 2 or 3, wherein the molar ratio of the base to the saccharide units is 1:1 to 10:1.

## Revendications

1. Procédé de préparation d'alkylglycosides par alkylation de saccharides réducteurs avec des agents d'alkylation en présence d'une base et d'un solvant, caractérisé en ce que l'on utilise, en tant qu'agent d'alkylation, des sulfates de dialkyle dont les groupements alkyle comportent au moins 6 atomes de carbone, et en ce que le rapport molaire des saccharides aux sulfates de dialkyle est compris entre 1:1 et 1:5.

2. Procédé selon la revendication 1, caractérisé en ce que les groupements alkyle des sulfates de dialkyle comportent de 6 à 30 atomes de carbone et contiennent éventuellement une double liaison.

3. Procédé selon la revendication 1 ou 2, caractérisé en ce que l'on utilise, comme base, les hydroxydes, les amides, les hydrures, les oxydes ou les carbonates de métaux alcalins et/ou de métaux alcalinoterreux, l'hydrure de bore, l'hydrure de sodium et de bore ou les hydrures, hydroxydes, oxydes ou carbonates de l'aluminium.

4. Procédé selon l'une quelconque des revendications 1 à 3, caractérisé en ce que le rapport molaire des bases utilisées aux motifs saccharide est compris entre 1:1 et 10:1.
